# EUROPÄISCHE PATENTSCHRIFT

(11) **EP 2 758 804 B1**
(45) Veröffentlichungstag und Bekanntmachung des Hinweises auf die Patenterteilung: **06.11.2019**
(21) Anmeldenummer: 11761332.3
(22) Anmeldetag: 20.09.2011
(51) Int. Cl.: G01S 13/06, G01S 17/06, G01S 19/19, G01S 5/00, A63B 24/00, G09B 19/00

(54) **SYSTEM UND VERFAHREN ZUM ERFASSEN EINES BENUTZERABHÄNGIGEN ZUSTANDES EINES SPORTGEGENSTANDES**
SYSTEM AND METHOD FOR DETECTING A USER-DEPENDENT STATE OF A SPORTS OBJECT
SYSTÈME ET PROCÉDÉ POUR DÉTECTER UN ÉTAT DÉPENDANT DE L'UTILISATEUR D'UN ARTICLE DE SPORT

(43) Veröffentlichungstag der Anmeldung: 30.07.2014
(73) Patentinhaber: Fraunhofer Gesellschaft zur Förderung der angewandten Forschung e.V., 80686 München (DE)
(72) Erfinder: LOCHMANN, Matthias, 91077 Neunkirchen am Brand (DE)
(74) Vertreter: Patentship Patentanwaltsgesellschaft mbH
(86) Internationale Anmeldenummer: PCT/EP2011/066264
(87) Internationale Veröffentlichungsnummer: WO 2013/041124

(56) Entgegenhaltungen:
- EP-A2- 2 025 372
- WO-A1-01/10517
- WO-A1-02/14894
- WO-A1-99/49279
- WO-A1-2010/065886
- US-A1- 2005 259 002

## Beschreibung

Die vorliegende Erfindung betrifft ein System und Verfahren zum Erfassen eines benutzerabhängigen Zustandes eines Sportgegenstandes.

Die menschliche Bewegung ist ein komplexer biomechanischer Ablauf. So muss beispielsweise ein Sportler, welcher einen Sportgegenstand, wie etwa einen Ball oder ein Wurfgerät verwendet, im Rahmen eines Trainings diejenigen Bewegungsabläufe einüben, welche zu einem optimalen Sportergebnis führen. In diesem Zusammenhang ist es auch wichtig zu erfahren, ob der Sportler den Spielgegenstand in einen bestimmten Bewegungszustand, wie etwa Ballabspielgeschwindigkeit, versetzen kann.

In WO 2010/065886 A1 ist ein Überwachungssystem zum Überwachen einer Leistungsfähigkeit eines Athleten offenbart. Das System umfasst einen Ball, in welchem ein Sensorsystem integriert ist.

In WO 01/10517 A1 ist ein System zum Bestimmen von Informationen offenbart, welche zu einer Bewertung dahingehend verwendet werden können, ob ein Spiel in Übereinstimmung mit Spielregeln gespielt wird.

In EP 2 025 372 A2 ist ein System zum Verfolgen von Sportbällen offenbart. Ein Sportball umfasst zwei Sender, welche Signale mit unterschiedlicher Frequenz aussenden.

In US 2005/0259002 A1 ist eine Vorrichtung zum Verfolgen einer Position eines Sportobjektes unter Verwendung eines Global-Positioning-Systems (GPS) offenbart.

In WO 02/14894 A1 ist eine Vorrichtung zur Bestimmung von Positionsdaten von beweglichen Objekten zur Auswertung von Ereignissen und/oder Relationen zwischen den beweglichen Objekten offenbart.

In WO 99/49279 A1 ist ein tragbares Rückmeldesystem zum regelmäßigen Bereitstellen von Aktualisierungen hinsichtlich einer Leistungsfähigkeit eines Athleten offenbart.

Es ist eine Aufgabe der vorliegenden Erfindung, ein Konzept zur Erfassung eines benutzerabhängigen Zustandes eines Sportgegenstandes zu schaffen.

Diese Aufgabe wird durch die Merkmale der unabhängigen Ansprüche gelöst. Vorteilhafte Weiterbildungsformen sind Gegenstand der abhängigen Ansprüche, der Beschreibung sowie der Figuren.

Die vorliegende Erfindung basiert auf der Erkenntnis, dass die obige Aufgabe durch eine Beobachtung des Spielgegenstandes gelöst werden kann. So kann beispielsweise anhand einer Mehrzahl von beobachteten Positionen eines Balls innerhalb eines vorbestimmten Zeitintervalls die Ballgeschwindigkeit bzw. die Ballbeschleunigung oder die Ballverweildauer bei einem Sportler erfasst werden.

Gemäß einem ersten Aspekt betrifft die Erfindung ein System zum Erfassen eines benutzerabhängigen Zustandes eines Sportgegenstandes mit einer Erfassungseinrichtung zum Erfassen einer Mehrzahl von Positionen des Sportgegenstandes und einer Bestimmungseinrichtung zum Bestimmen des Zustandes des Sportgegenstandes auf der Basis der erfassten Mehrzahl der Positionen.

Gemäß einer Ausführungsform ist der Sportgegenstand ein Ball oder ein Puck. Der Benutzer ist ein Sportler.

Gemäß einer Ausführungsform ist der Zustand des Sportgegenstandes einer der folgenden Zustände: Verweildauer des Sportgegenstandes bei einem Benutzer, Geschwindigkeit, insbesondere translatorische Geschwindigkeit oder Rotationsgeschwindigkeit des Sportgegenstandes, Impuls, insbesondere Drehimpuls oder translatorischer Impuls des Sportgegenstandes, Bewegungsrichtung des Sportgegenstandes, Beschleunigung, insbesondere positive oder negative Beschleunigung des Sportgegenstandes.

Gemäß einer Ausführungsform ist der Sportgegenstand ausgebildet ein Positionssignal, insbesondere ein Global Positioning Signal, auszusenden, und wobei die Erfassungseinrichtung ausgebildet ist, das Positionssignal zu empfangen, um die Mehrzahl der Positionen zu bestimmen. Die Erfassungseinrichtung ist ausgebildet, das Positionssignal zu empfangen und die Mehrzahl der Positionen des Spielgegenstandes auf der Basis des empfangenen Positionssignals zu bestimmen. Zum Empfang des Positionssignals kann die Erfassungseinrichtung eine Empfangsantenne umfassen, welche das Positionssignal empfängt. Die Erfassungseinrichtung kann ferner ausgebildet sein, das empfangene Positionssignal zu verarbeiten, um die Positionen des Spielgegenstands zu erfassen.

Gemäß einer Ausführungsform ist die Erfassungseinrichtung ausgebildet, ein an dem Sportgegenstand reflektiertes Reflexionssignal, insbesondere ein Radarsignal oder ein Lasersignal, zu empfangen, und auf der Basis des reflektierten Reflexionssignals die Mehrzahl der Positionen zu bestimmen.

Gemäß einer Ausführungsform ist die Erfassungseinrichtung ausgebildet, ein Sendesignal, insbesondere ein Radarsignal oder ein Lasersignal, auszusenden, um das Reflexionssignal zu erzeugen. Der Spielgegenstand kann hierzu beispielsweise eine reflektierende Oberfläche aufweisen, welche metallisch sein kann, um ein Radarsignal zu reflektieren.

Gemäß einer Ausführungsform ist die Bestimmungseinrichtung ausgebildet, einen ersten Zeitpunkt einer Annahme des Sportgegenstandes durch den Benutzer und einen zweiten Zeitpunkt einer Abgabe des Sportgegenstandes durch den Benutzer zu bestimmen, um eine Verweildauer des Sportgegenstandes bei dem Benutzer als den benutzerabhängigen Zustand des Sportgegenstandes zu bestimmen.

Gemäß einer Ausführungsform ist die Bestimmungseinrichtung ausgebildet, einen dritten Zeitpunkt eines Umlegens des Sportgegenstandes durch den Benutzer zwischen dem ersten Zeitpunkt und dem zweiten Zeitpunkt zu bestimmen, um eine erste Unterverweildauer zwischen dem ersten und dem dritten Zeitpunkt und eine zweite Unterverweildauer zwischen dem dritten und dem zweiten Zeitpunkt zu bestimmen. Der dritte Zeitpunkt als ein weiterer benutzerabhängiger Zustand kann beispielsweise dadurch bestimmt werden, dass ein Spieler den Ball von dem einen auf den anderen Fuß umlegt.

Gemäß einer Ausführungsform ist die Bestimmungseinrichtung ausgebildet, zur Bestimmung des benutzerabhängigen Zustandes einen Zeitpunkt einer Annahme des Sportgegenstandes durch den Benutzer durch eine Erfassung einer negativen Beschleunigung des Sportgegenstandes anhand eines zeitlichen Verlaufes der Mehrzahl der Positionen des Sportgegenstandes zu bestimmen.

Gemäß einer Ausführungsform ist die Bestimmungseinrichtung ausgebildet, zur Bestimmung des benutzerabhängigen Zustandes einen Zeitpunkt einer Abgabe des Sportgegenstandes durch eine Erfassung einer positiven Beschleunigung des Sportgegenstandes anhand eines zeitlichen Verlaufes der Mehrzahl der Positionen des Sportgegenstandes zu bestimmen.

Gemäß einer Ausführungsform umfasst das System einen Sportgegenstandgeber, insbesondere einen Ballgeber, zum Ausgeben des Sportgegenstandes.

Gemäß einer Ausführungsform umfasst das System einen Sportgegenstandfänger zum Fangen des Sportgegenstandes nach einer Sportgegenstandabgabe durch den Benutzer.

Gemäß einer Ausführungsform ist der Sportgegenstandfänger ein Balltor oder eine Sensorwand.

Gemäß einer Ausführungsform ist die Erfassungseinrichtung ausgebildet, die erfasste Mehrzahl der Positionen zu speichern.

Gemäß einer Ausführungsform umfasst das System eine Anzeigeeinrichtung zum Anzeigen der erfassten Mehrzahl der Positionen oder zum Projizieren eines Projektionsbereiches auf ein Spielfeld, insbesondere um einen weiteren Spieler herum.

Gemäß einer Ausführungsform ist die Bestimmungseinrichtung ausgebildet, den benutzerabhängigen Zustand mit einem Referenzzustand zu vergleichen, um den benutzerabhängigen Zustand zu bewerten.

Gemäß einer Ausführungsform ist die Erfassungseinrichtung zum Erfassen einer Ist-Position des Spielgegenstandes ausgebildet und die Bestimmungseinrichtung ist zum Bestimmen einer Soll-Position des Spielgegenstandes ausgebildet. Die Anzeigeeinrichtung ist zum Anzeigen eines Hinweises auf die Soll-Position ausgebildet, wenn sich die Ist-Position von der Soll-Position unterscheidet.

Der Hinweis auf die Soll-Position kann eine Anzeige der Soll-Position selbst oder eine Anzeige, dass die Soll-Position noch nicht erreicht wurde, oder eine Anzeige einer Richtung der Soll-Position umfassen. Zur Feststellung, ob sich die Ist-Position von der Soll-Position unterscheidet, kann die Bestimmungseinrichtung die Ist-Position mit der Soll-Position vergleichen. Hierbei können die Ist-Position und die Soll-Position in der Gestalt von digitalen Positionsdaten vorliegen.

Gemäß einer Ausführungsform ist die Bestimmungseinrichtung ausgebildet, eine vorbestimmte Position des Spielgegenstandes, insbesondere eine vorgebbare Position des Spielgegenstandes, als die Soll-Position zu bestimmen. Die vorbestimmte Position des Spielgegenstandes kann beispielsweise aus einer Mehrzahl von möglichen Soll-Positionen ausgewählt werden. Diese Auswahl kann zufällig oder deterministisch sein. Die vorbestimmte, deterministische Position kann beispielsweise anhand einer Regel vorgegeben werden.

Gemäß einer Ausführungsform ist der Spielgegenstand ein Ball, und die Bestimmungseinrichtung ist ausgebildet, einen Sensorbereich einer Sensorwand als die Soll-Position zu bestimmen, insbesondere auszuwählen. Gemäß einer Ausführungsform ist die Sensorwand ein Element des Systems. Gemäß einer anderen Ausführungsform ist die Sensorwand kein Element des Systems. Die Sensorwand kann zumindest einen Sensor, beispielsweise einen Drucksensor, zur Detektion des Balls aufweisen. Die Sensorwand kann gemäß einer Ausführungsform jedoch derart gebildet sein, dass das Eintreffen des Balls durch beispielsweise eine Einwölbung oder Auswölbung der Sensorwand beispielsweise optisch detektierbar ist.

Auf diese Weise kann beispielsweise einem Fußballspieler als Soll-Position des Balls ein Bereich der Sensorwand angezeigt werden, welcher ein Tor darstellt. Der Fußballspieler kann auf diese Weise rasch unterschiedliche Ballschießübungen ausführen.

Gemäß einer Ausführungsform kann das System einen oder mehrere Ballgeber umfassen, welcher einen Spielgegenstand, beispielsweise einen Ball, abgibt. Der Ballgeber kann ausgebildet sein, den Ball in eine vorbestimmte Richtung, beispielsweise zum Spieler hin, abzugeben. Die Anzeigeeinrichtung kann durch die Sensorwand gebildet sein, welche den Sensorbereich als die Soll-Position anzeigt.

Gemäß einer Ausführungsform ist die Bestimmungseinrichtung ausgebildet, die Soll-Position in Abhängigkeit von der Ist-Position des Spielgegenstandes zu bestimmen. Auf diese Weise wird eine dynamische Bestimmung der Soll-Position in Abhängigkeit von beispielsweise einem Spielgeschehen auf einem Spielfeld ermöglicht.

Gemäß einer Ausführungsform ist die Bestimmungseinrichtung ausgebildet, die Soll-Position in Abhängigkeit von der Ist-Position des Spielgegenstandes und einer geometrischen Charakteristik eines Bereichs, insbesondere eines Spielfeldes, innerhalb dessen der Spielgegenstand bewegbar ist, oder die Soll-Position in Abhängigkeit von der Ist-Position des Spielgegenstandes bezüglich einer geometrischen Charakteristik des Spielfeldes, insbesondere einer Tormitte, zu bestimmen. So kann beispielsweise die Soll-Position als eine Verbindungslinie zwischen Tormitte und Ball in Abhängigkeit von einer Ballposition bestimmt werden.

Gemäß einer Ausführungsform ist die Bestimmungseinrichtung ausgebildet, die Soll-Position als eine Abfolge sukzessiver Hilfs-Positionen zu bestimmen. Durch die sukzessive Abfolge der Hilfspositionen wird das schrittweise Erreichen einer endgültigen Soll-Position vereinfacht, da beispielsweise kleinere Bewegungsschritte geübt werden können.

Gemäß einer Ausführungsform ist die Erfassungseinrichtung ausgebildet, eine weitere Ist-Positionen von einem weiteren Spielgegenstand zu bestimmen, und die Bestimmungseinrichtung ist ausgebildet, die Soll-Position des Spielgegenstandes in Abhängigkeit von der weiteren Ist-Position, insbesondere relativ zu der weiteren Ist-Position des weiteren Spielgegenstandes zu bestimmen.

Gemäß einer Ausführungsform ist die Bestimmungseinrichtung ausgebildet, die Soll-Position anhand einer vorgegeben Regel, welche die Soll-Position mit einer Ist-Position verknüpft, zu bestimmen. Die Regel kann beispielsweise die Soll-Position eines Spielers bezüglich der weiteren Ist-Position eines weiteren Spielers verknüpfen.

Gemäß einer Ausführungsform ist die Erfassungseinrichtung ausgebildet, eine Mehrzahl von weiteren Ist-Positionen von einer Mehrzahl von weiteren Spielgegenständen zu bestimmen, und die Bestimmungseinrichtung ist ausgebildet, die Soll-Position des Spielgegenstandes in Abhängigkeit von der Mehrzahl der weiteren Ist-Positionen zu bestimmen. Die Mehrzahl der weiteren Ist-Positionen kann beispielsweise durch Ist-Positionen von Spielern einer Spielmannschaft bestimmt werden. Auf diese Weise kann die Soll-Position eines einzelnen Spielers bezüglich der Spielmannschaft bestimmt und/oder angezeigt werden.

Gemäß einer Ausführungsform ist die Bestimmungseinrichtung ausgebildet, einen Schwerpunkt, insbesondere einen geometrischen oder gewichteten Schwerpunkt, der weiteren Ist-Positionen zu bestimmen, und die Soll-Position in Abhängigkeit von dem Schwerpunkt, insbesondere relativ zu dem Schwerpunkt, zu bestimmen. Der geometrische Schwerpunkt kann beispielsweise anhand jedes an sich bekannten Algorithmus bestimmt werden, welcher eine Bestimmung eines geometrischen Schwerpunktes ermöglicht. Der gewichtete Schwerpunkt kann beispielsweise einen geometrischen Schwerpunkt von gemäß einer Ausführungsform gewichteten Ist-Positionen der weiteren Spielgegenstände sein. Bei der Wichtung kann beispielsweise die Bedeutung eines Spielers, beispielsweise eines Torwarts, für eine bestimmte Spielsituation zum Ausdruck gebracht werden.

Gemäß einer Ausführungsform ist die Bestimmungseinrichtung ausgebildet, die Soll-Position in Abhängigkeit von dem geometrischen Schwerpunkt anhand einer vorgegebenen Regel, welche Soll-Positionen mit geometrischen Schwerpunkten verknüpft, zu bestimmen. Diese Regel kann beispielsweise anhand von Erfahrungswerten erstellt werden.

Gemäß einer Ausführungsform ist die Anzeigeeinrichtung ausgebildet, als Hinweis auf die Soll-Position die Soll-Position selbst oder einen Hinweis auf die Lage der Soll-Position bezüglich einer Lage des Spielgegenstandes, insbesondere auf eine Richtung zu der Soll-Position, oder als einen Hinweis auf eine Differenz zwischen der Ist-Position und der Soll-Position anzuzeigen. Die Differenz kann beispielsweise durch ein akustisches Signal angezeigt werden, beispielsweise eine Schwebung oder ein in der Frequenz veränderliches Signal, wobei die Schwebungsfrequenz oder die Signalfrequenz unmittelbar von der Differenz abhängen.

Gemäß einer Ausführungsform ist die Anzeigeeinrichtung ausgebildet, den Hinweis auf die Soll-Position akustisch, optisch, akusto-optisch, taktil, insbesondere mittels einer Vibration oder eines Drucks, anzuzeigen. Die Anzeigevorrichtung kann hierzu durch beispielsweise den Benutzer tragbar sein, um ein beispielsweise taktiles Signal zu erzeugen, das dem Benutzer, beispielsweise Spieler, den Hinweis auf die Soll-Position anzeigt. Die Anzeigeeinrichtung kann jedoch einen Bildschirm umfassen oder ausgebildet sein, den Hinweis durch die Projektion auf eine Projektionsfläche, beispielsweise auf ein Spielfeld oder auf eine Visierscheibe eines Helmvisiers, zu projizieren.

Gemäß einer Ausführungsform ist der Spielgegenstand ein Spielball, insbesondere ein Fußball oder ein Tischtennisball oder ein Tennisball oder ein Rugbyball, oder ein Puck, und die Anzeigeeinrichtung umfasst eine Sensorwand zum Detektieren des auf die Sensorwand eintreffenden Spielgegenstandes, und die Anzeigeeinrichtung ist ausgebildet, einen Bereich der Sensorwand als Hinweis auf die Soll-Position durch visuelles Hervorheben, insbesondere durch Leuchten oder Beleuchten des Bereichs, oder durch akustisches Hervorheben anzuzeigen. Die Sensorwand kann beispielsweise Merkmale der vorgenannten Sensorwand aufweisen oder der vorgenannten Sensorwand entsprechen.

Gemäß einer Ausführungsform ist die Anzeigeeinrichtung ausgebildet, den Hinweis auf die Soll-Position auf einem elektronischen Display anzuzeigen. Die Bestimmungseinrichtung kann ausgebildet sein, das Display geeignet anzusteuern.

Gemäß einer Ausführungsform ist die Anzeigeeinrichtung ausgebildet, das Display eines Smartphones zum Anzeigen des Hinweises auf die Soll-Position anzusteuern.

Gemäß einer Ausführungsform können sowohl die Erfassungseinrichtung und/oder die Bestimmungseinrichtung und/oder die Anzeigeeinrichtung auf einem derartigen Smartphone beispielsweise in Software mittels eines Applikationsprogramms realisiert werden.

Gemäß einer Ausführungsform ist die Anzeigeeinrichtung ausgebildet, den Hinweis auf die Soll-Position auf ein Spielfeld mittels eines Lichts zu projizieren, insbesondere mittels einer Laser-Projektion oder einer LED-Projektion zu projizieren.

Gemäß einer Ausführungsform ist die Bestimmungseinrichtung ausgebildet, die Soll-Position in Abhängigkeit von einem Körperparameter, insbesondere Herzfrequenz, Herzfrequenzvariabilität, Atemfrequenz, Körpertemperatur, Blutwertparameter wie Zuckerkonzentration oder Sauerstoffkonzentration, zu bestimmen. Die Körperparameter können beispielsweise mittels berührungsloser Sensoren ermittelt und zum Beispiel an die Erfassungseinrichtung ausgesendet werden.

Gemäß einer Ausführungsform umfasst die Bestimmungseinrichtung zumindest eine Positionsbestimmungseinrichtung, insbesondere einen Positionssender, zur Erfassung der Ist-Position. Ein derartiger Positionssender kann beispielsweise an einem Spieler oder in einem Ball untergebracht werden. Generell ermöglicht diese Ausführungsform, dass das erfindungsgemäße System durch einen Benutzer getragen werden kann.

Gemäß einer Ausführungsform umfasst das System eine Mehrzahl der Erfassungseinrichtungen zur Erfassung der Ist-Position.

Gemäß einem weiteren Aspekt betrifft Erfindung ein Verfahren zum Erfassen eines benutzerabhängigen Zustandes eines Sportgegenstandes, mit Erfassen einer Mehrzahl von Positionen des Sportgegenstandes, und Bestimmen des Zustandes des Sportgegenstandes auf der Basis der erfassten Mehrzahl der Positionen.

Weitere Merkmale des Verfahrens ergeben sich unmittelbar aus der Funktionalität des Systems oder eines Merkmals des Systems.

Gemäß einer Ausführungsform kann das Verfahren durch das System ausgeführt werden.

Gemäß einem weiteren Aspekt betrifft die Erfindung ein Computerprogramm mit einem Programmcode zum Ausführen des erfindungsgemäßen Verfahrens, wenn der Programmcode auf einem Computer ausgeführt wird.

Weitere Ausführungsbeispiele der Erfindung werden Bezug nehmend auf die beiliegenden Zeichnungen näher erläutert. Es zeigen:
- Fig. 1: ein Blockdiagramm eines Systems zur Unterstützung einer Bewegungsübung mit einem Spielgegenstand gemäß einer Ausführungsform;
- Fig. 2: ein System zur Unterstützung einer Bewegungsübung gemäß einer Ausführungsform;
- Fig. 3: ein System zur Unterstützung einer Bewegungsübung gemäß einer weiteren Ausführungsform;
- Fig. 4: ein System zur Unterstützung einer Bewegungsübung gemäß einer weiteren Ausführungsform;
- Fig. 5: ein System zur Unterstützung einer Bewegungsübung gemäß einer weiteren Ausführungsform;
- Fig. 6: ein System zur Unterstützung einer Bewegungsübung gemäß einer weiteren Ausführungsform; und
- Fig. 7: ein System zur Unterstützung einer Bewegungsübung gemäß einer weiteren Ausführungsform.

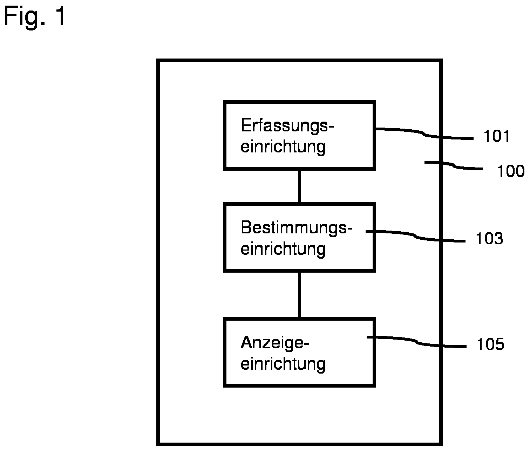

Fig. 1 zeigt ein Blockdiagramm eines Systems 100 zum Erfassen eines benutzerabhängigen Zustandes eines Sportgegenstandes. Das System 100 umfasst eine Erfassungseinrichtung 101 zum Erfassen einer Mehrzahl von Positionen des Sportgegenstandes und eine Bestimmungseinrichtung 103 zum Bestimmen des Zustandes des Sportgegenstandes auf der Basis der erfassten Mehrzahl der Positionen.

Der Zustand des Sportgegenstandes kann einer der folgenden Zustände sein Verweildauer des Sportgegenstandes bei einem Benutzer, Geschwindigkeit, insbesondere translatorische Geschwindigkeit oder Rotationsgeschwindigkeit des Sportgegenstandes, Impuls, insbesondere Drehimpuls oder translatorischer Impuls des Sportgegenstandes, Bewegungsrichtung des Sportgegenstandes, Beschleunigung, insbesondere positive oder negative Beschleunigung des Sportgegenstandes.

Optional kann das System eine Anzeigeeinrichtung 105 umfassen. Die Anzeigeeinrichtung 105 kann vorgesehen sein, die erfasste Mehrzahl der Positionen, beispielsweise auf einem Display, anzuzeigen.

Alternativ oder zusätzlich kann die Anzeigeeinrichtung 105 ausgebildet sein, einen Projektionsbereich auf ein Spielfeld zu projizieren/ zu werfen. Handelt es sich bei dem Sportgegenstand um einen Ball und bei Zustand des Sportgegenstandes um eine Verweildauer des Ball bei einem Spieler auf einem Spielfeld, so kann die Anzeigeeinrichtung 105 ausgebildet sein, einen Projektionsbereich, beispielsweise einen Kreis, um einen weiteren Spieler herum auf das Spielfeld zu projizieren, um dem weiteren Spieler anzuzeigen, dass er innerhalb des Projektionsbereichs bleiben soll. Die Projektion kann nach einer vorbestimmten Zeit der Verweildauer, beispielsweise nach einer Verweildauer von 1 Sekunde oder 2 Sekunden, beendet sein, um dem weiteren Spieler anzuzeigen, dass er den Spieler angreifen kann. Dadurch kann eine reale Spielsituation simuliert werden.

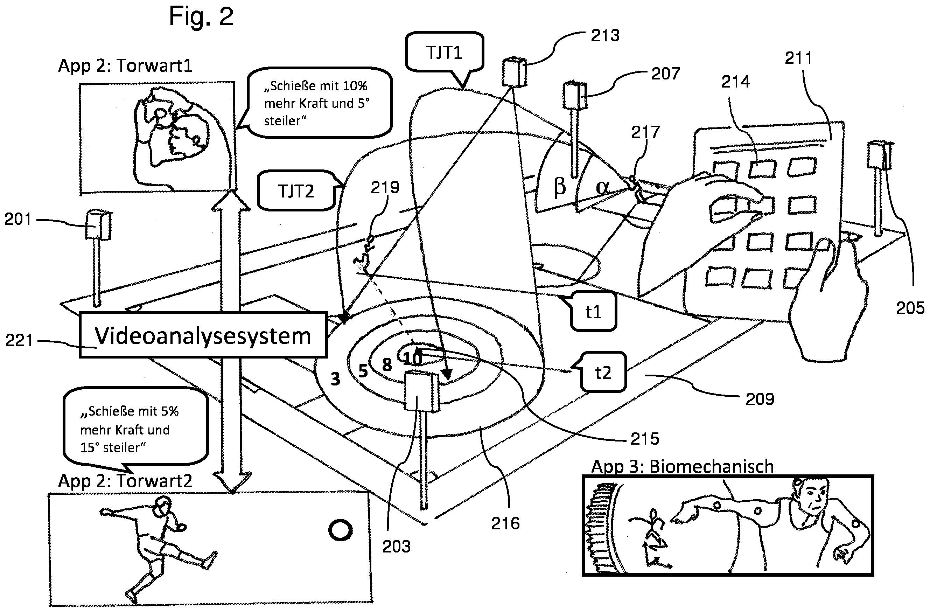

Fig. 2 zeigt ein System zur Unterstützung einer Bewegungsübung mit einem Spielgegenstand, der beispielsweise ein Fußball ist. Das System umfasst ein Positionslokalisierungssystem mit einer Mehrzahl von Erfassungseinrichtungen 201, 203, 205 und 207 (PLS1, PLS2, PLS3, PLS4), welche beabstandet voneinander, beispielsweise in Ecken eines Fußballfeldes 209, angeordnet sind. Das System kann jedoch nur eine, zwei oder drei oder mehr als 4 Erfassungseinrichtungen umfassen.

Das System umfasst ferner eine Bestimmungseinrichtung 211 zum Bestimmen einer Ist-Position des Spielgegenstandes, welche der gegenwärtigen Ist-Position des Spielgegenstandes entspricht. Die Bestimmungseinrichtung kann beispielsweise auf einem Smartphone mittels eines Applikationsprogramms realisiert werden, mit mehreren Applikationsfeldern 214, welche jeweils eine Auswahl einer Soll-Position ermöglichen. Die Bestimmungseinrichtung 211 kann jedoch auch ein separater Rechner oder ein Rechnercluster sein.

Das System umfasst ferner eine Anzeigeeinrichtung 213 zum Anzeigen eines Hinweises auf die Soll-Position, sofern sich die Ist-Position von der Soll-Position unterscheidet. Die Soll-Position 215 kann beispielsweise in einem Bereich 216 des Spielfeldes 209 liegen. Die Anzeigevorrichtung 213 kann beispielsweise ausgebildet sein, konzentrische Kreise um die Soll-Position 215 auf das Spielfeld zu projizieren, um beispielsweise einem Torwart 217 die Soll-Position anzuzeigen. Die Ist-Position des Spielgegenstandes kann beispielsweise der Ist-Position des Torwarts 217 entsprechen. Befindet sich beispielsweise auf dem Spielfeld 209 ein weiterer Spieler 219, so kann die Soll-Position 215 in Abhängigkeit von einer Ist-Position des weiteren Spielers 219 bestimmt und/oder angezeigt werden. Hierzu kann der weitere Spieler 219 mit einem Sender ausgestattet sein, welcher eine Position des weiteren Spielers 219 an die Erfassungseinrichtungen 201 bis 207, welche eine gemeinsame Erfassungseinrichtung bilden, aussendet.

Das System kann ferner ein Videoanalysesystem 221 umfassen, dass als Applikation das Verhalten eines ersten Torwarts und eines zweiten Torwarts anzeigen kann.

Um mit einem weiten Abschlag aus der Hand zielgenau einen Ball in den Lauf eines Angreifers spielen zu können sind verschiedene Teilfertigkeiten eines Torhüters (TW1) vorteilhaft. Zunächst ist dies die Antizipationsfähigkeit des Torhüters im Hinblick auf die zu erwartende Position eines Mitspielers zum Zeitpunkt der Ballannahme durch den Mitspieler P1 in Position 10. Weiterhin sollte der Torhüter in Bezug auf sein technisches, Kraft- und Koordinationsniveau dazu in der Lage sein, den Ball adäquat mit folgenden biomechanischen Einflussgrößen anzusteuern:
- Linearen Impuls
- Drehimpuls (links, rechts)
- Abschlagwinkel (horizontal, vertikal)

Die hier genannten biomechanischen Einflussgrößen sind ebenso wie die Torhüterbewegung in Echtzeit in an sich bekannter Weise erfassbar.

Beginnt nun der anzuspielende Spieler P1 zum Zeitpunkt t1 mit der mittleren Geschwindigkeit v in eine bestimmte Richtung zu laufen, so kann das System basierend auf Modellannahmen, welche deterministische biomechanische Gesetzmäßigkeiten umfassen und in der Datenbank hinterlegt sein können, vorausberechnen wo der Ball zum Zeitpunkt t2 landen würde, wenn er beispielsweise mit vorbekannten biomechanischen Eingangsvoraussetzungen abgeschlagen wird um zum Zeitpunkt t2 in der Nähe des Fußes des Spielers zu landen. Dieses anzusteuernde Ziel in der Gestalt einer Zielscheibe kann permanent oder dynamisch veränderbar von der Anzeigeeinrichtung 213 auf die Spiel- bzw. Trainingsfläche projiziert werden. Der Torhüter könnte somit schon bevor er den Ball final abschlägt per Echtzeitfeedback die Zielzone visualisiert bekommen. Ebenso stehen in geringer Zeitverzögerung (0,1 s-0,5 s) jene Parameter zur Verfügung unter deren Einfluss die Balltrajektorie zu Stande gekommen ist, mit Abschlagwinkel alpha bzw. beta, Impuls des Balles p, Balltrajektorie, etc. In Echtzeit kann mit dem System auch die Zielpräzision des Abschlages angegeben werden, beispielsweise innerhalb der Zielscheibe. Im Lern- bzw. Echtzeitrückkopplungsmodus kann der Torhüter nun entscheiden auf welcher Grundlage die Rückkopplung erfolgen soll. Hierzu wählt der Torhüter oder Trainer zwischen verschiedenen Applikation (Apps) auf einem Tablet-PC, PC, Notebook, Smartphone oder sonstigem Anzeigegerät aus. Er kann entscheiden ob die Soll/ Istwert Abweichung auf der Basis des statistischen Datenmaterials das die Datenbank über einen Torhüter über den die Datenbank Daten enthält erfolgt. Alternativ kann er sich auch in Referenz zu unterschiedlichen biomechanischen Modellen in eine Echtzeit-Trainings- und Lerneinheit begeben. Er kann dann auswählen, ob die Ausführung des Abschlages weitenoptimiert, präzisionsoptimiert oder zeitoptimiert erfolgen soll.

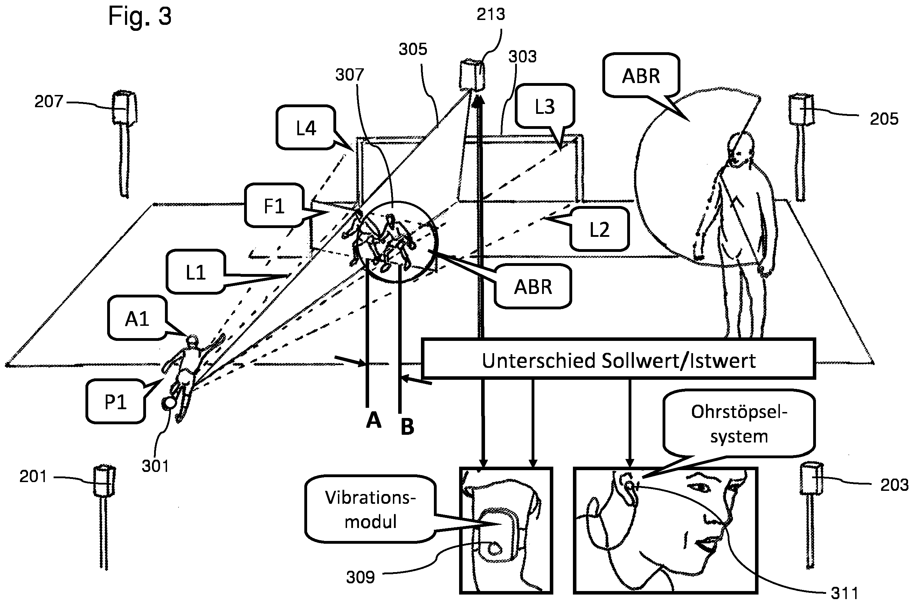

Fig. 3 zeigt ein System zur Unterstützung einer Bewegungsausübung mit einem Spielgegenstand, beispielsweise mit einem Fußball, gemäß einer Ausführungsform. Das System umfasst zumindest eine der in Fig. 2 dargestellten Erfassungseinrichtungen 201 bis 207. Diese erfassen beispielsweise eine Ist-Position eines Spielgegenstandes 301, welches ein Ball sein kann. Die Bestimmungseinrichtung, welche in Fig. 3 nicht dargestellt ist, ermittelt beispielsweise anhand der Ist-Position des Balls 301 und der Mitte eines Tors 303 eine Verbindungslinie 305 zwischen Tormitte und der Ist-Position des Balls 301.

Die Anzeigeeinrichtung 213 kann ausgebildet sein, die Verbindungslinie 305 auf das Spielfeld zu projizieren und einem Torwart 307, welcher gemäß einer Ausführungsform ein Spielgegenstand im Sinne der vorliegenden Beschreibung sein kann, als Soll-Position anzuzeigen.

Gemäß einer Ausführungsform kann die Anzeigeeinrichtung 213 alternativ oder zusätzlich zumindest ein Vibrationsmodul 309 umfassen, welches dem Torwart die Richtung zur Verbindungslinie 305 anzeigt. Hierzu kann der Torwart beidseitig, beispielsweise an den Oberarmen, Vibrationsmodule tragen, welche in Abhängigkeit von der Ist-Position des Torwarts 307 einen Vibrationshinweis in Richtung der Verbindungslinie 305 anzeigen.

Gemäß einer Ausführungsform kann die Anzeigevorrichtung 213 alternativ oder zusätzlich zumindest einen Lautsprecher 311 umfassen, welcher beispielsweise in der Gestalt eines Ohrsteckers, beispielsweise am Ohr des Torwarts, befestigt werden kann. Der Lautsprecher 311 ist ausgebildet, akustische Signale auszugeben, welche auf die Lage der Soll-Position, d.h. welche auf die Lage der Verbindungslinie 305 hinweisen. Gemäß einer Ausführungsform kann so auf die Projektion der Verbindungslinie auf das Spielfeld verzichtet werden. Gemäß einer anderen Ausführungsform können zumindest zwei der vorgenannten Ausführungsformen, beispielsweise Projektion und Vibration oder Projektion und akustisches Signal, gemeinsam eingesetzt werden.

Schießt beispielweise der Angreifer A1 aus der Position P1 aufs Tor, so geht bei einer annähernd geradlinigen Ballflugbahn der Ball aus geometrischen Gründen nur dann ins Tor, wenn er sich innerhalb eines Raumes bewegt, der sich zwischen dem Ball und den gedachten Verbindungslinien (L1-L4) aufspannt. Die individualtaktische Aufgabe des Torhüters besteht nun darin durch seine Körperhaltung und Position mit seiner anthropometrisch bedingten Reichweite (ABR) eine möglichst große Fläche der der Fläche (F1) abzudecken. Dies gelingt ihm dann am besten, wenn er auf der gedachten Linie zwischen Ballmitte und Tormitte agiert. Diese Linie kann mit der Anzeigeeinrichtung 213, welche ein optisch-akustisch-taktiles Feedbacksystem bilden kann, während des Schusstrainings bzw. Spiels auf das Spiel- bzw. Trainingsfeld projiziert werden. Da die Ortsposition des Balles 301 in Echtzeit an das System übermittelt werden kann, wandert diese Linie permanent mit der Ballposition mit. Der Torhüter kann somit via visuellem Echtzeitfeedback sein individualtaktisches Stellungsspiel permanent optimieren. Parallel zum visuellen Echtzeitfeedback erfolgt ein akustisches Feedback über ein Minikopfhörersystem und/oder ein kleines Lautsprechersystem, das am Körper des Torhüters befestigt ist und/oder über eine Beschallungsanlage. Dieses Audiofeedback funktioniert derart, dass beispielsweise bei der Einnahme der korrekten Position der Kammerton a eingespielt wird und bei größer werdender Soll-/Istwert Differenz (SID) die Frequenz proportional zur Entfernung von der Idealposition verändert wird. Ebenso könnte das System von der akustischen Rückmeldung funktionieren wie das Echolot oder wie eine akustische Rückmeldung einer Einparkhilfe, etc. Die dritte Komponente des Echtzeitfeedback erfolgt in Form einer Soll/Istwert Differenz gesteuerten Veränderung der Vibrationsfrequenz und/oder Amplitude eines taktilakustischen Feedbacksystems, das am Körper des Torhüters getragen werden und ein Element der Anzeigeeinrichtung 213 sein kann.

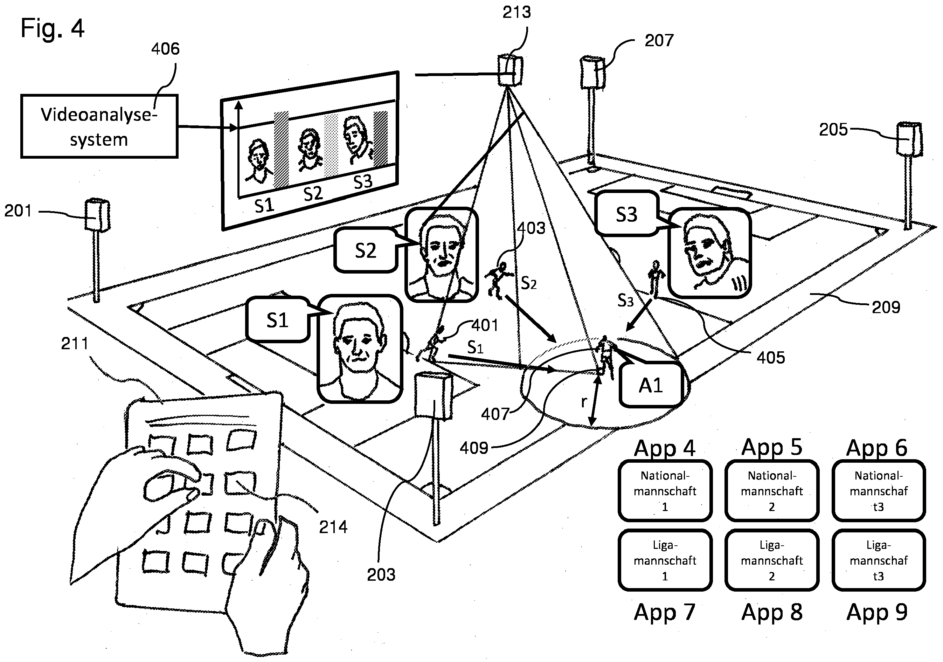

Fig. 4 zeigt ein System zur Unterstützung einer Bewegungsübung gemäß einer weiteren Ausführungsform. Die auf dem Spielfeld 209 befindlichen Spieler 401 (S1), 403 (S2), 405 (S3) und 407 (A1) führen eine Übung mit einem Ball 409 durch, welcher sich beispielsweise bei dem Spieler 407 befindet, der ein Angreifer ist. Die Spieler 401, 403 und 405 können beispielsweise als Spielgegenstande im Sinne der vorliegenden Beschreibung verstanden werden. Die Anzeigeeinrichtung 213 kann beispielsweise Signale erzeugen, welche den Spielern 401 bis 405 oder nur einem der Spieler 401, 403, 405 ein Signal übermitteln, welches einen Hinweis auf dessen Soll-Position enthält. Der Hinweis auf die Soll-Position kann dem jeweiligen Spieler 401, 403, 405 visuell, akustisch oder durch eine Vibration (taktil) angezeigt werden. Hierzu kann die Erfassungseinrichtung 213 beispielsweise ein Vibrationsmodul oder einen Lautsprecher aufweisen, welche an den jeweiligen Spieler 401 bis 405 angebracht sein können. Das System kann ferner ein Videoanalysesystem 406 umfassen.

In dem in Fig. 4 dargestellten Ausführungsbeispiel ist die Gruppentaktik gegen den Ball mit sogenanntem Pressing verdeutlicht.

Im modernen Fußball beginnt das verteidigen bereits tief in der gegnerischen Platzhälfte. Kommt z.B. ein Angreifer der gegnerischen Mannschaft (A1) in Ballbesitz, so besteht die Aufgabe der Ihn umgebenden Gegenspieler (S1-S3) darin, den Spieler A1 so aggressiv wie möglich zu attackieren um wieder selbst in Ballbesitz zu kommen. Dies nennt man Pressing. Die Aggressivität mit der ein Spieler presst ist durch die Höhe der Beschleunigung messbar und beurteilbar. Liegen nun in der Datenbank Referenzwerte bezüglich des Beschleunigungsverhaltens in Pressingsituationen der National- und Klubmannschaften und der weltbesten Fußballer vor, so können diese genutzt werden um als Sollwerte in einem Echtzeitfeedback - Training, -Coaching und -Learning eingesetzt werden zu können. Der Ablauf in einer Echtzeitfeedback-Trainingsituation wäre gemäß einer Ausführungsform der Folgende:
1) Ein Trainer wählt auf Tablet-PC, Smartphone, PC Notebook oder sonstiger Einstell- und Displayeinheit der Bestimmungseinrichtung 211 aus, mit welchen Referenzwerten aus der Datenbank der Abgleich erfolgen soll um zu den Soll-/Istwertdifferenzen zu gelangen. Die Apps 4,5,6 referenzieren auf die Normwerte von Nationalteams und die Apps 7,8,9 auf die Referenzwerte von Klubmannschaften.
2) Der Trainer hält beispielsweise einen Drucktaster in der Hand, mit dem er den Zeitpunkt zu dem Pressing von der Trainingsgruppe gespielt werden soll steuern kann. Drückt der Trainer diesen Taster, so wird durch die Anzeigeeinrichtung 213 ein Lichtkegel mit dem Radius r um den ballführenden Spieler projiziert, ebenso erfolgt ein Signalton über die Beschallungsanlage und/oder Minikopfhörer und/oder am Körper befindliche Lautsprechersysteme. Zusätzlich erfolgt je nach Setup auch eine taktile Rückmeldung über ein in Frequenz und Amplitude steuerbares Vibrationssystem.
3) Läuft nun einer der Spieler mit zu stark von den Referenzwerten abweichenden Beschleunigungswerten auf den ballführenden Gegenspieler, so kann dies wiederum durch die beschriebenen Echtzeitfeedbackmethoden an den Spieler zurückgemeldet werden.
4) Ist die Aktion vorüber, so kann das die Anzeigeeinrichtung 213 die Sollwerte, Istwerte und die Soll-Istwert Differenz auf eine Fläche projizieren und/oder das Ergebnis auf einem Tablet-PC und/oder einem Smartphone oder einer sonstigen Steuer- und Visualisierungseinheit anzeigen.

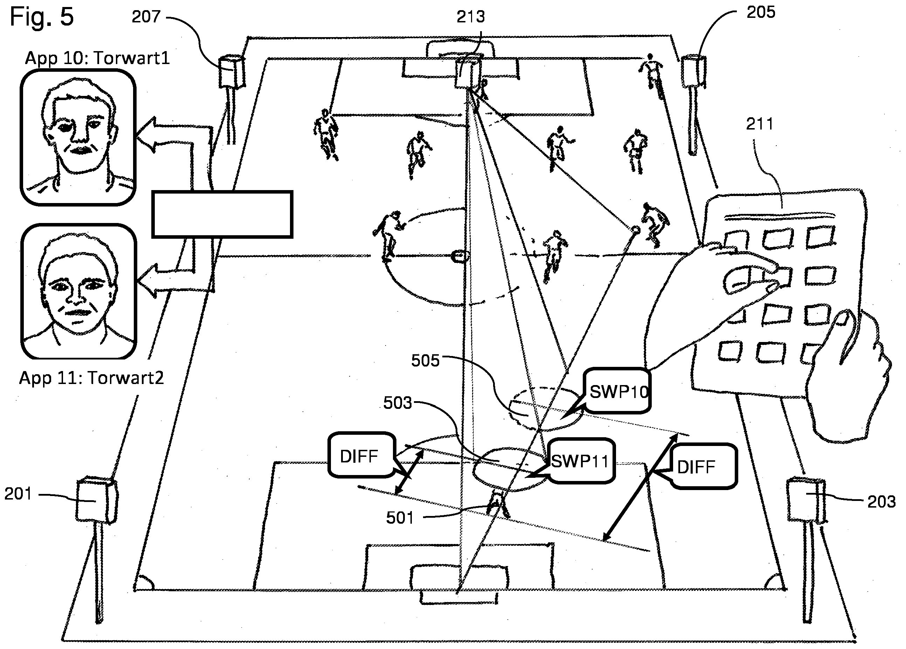

Fig. 5 zeigt ein System gemäß einer weiteren Ausführungsform, bei dem beispielsweise einem Torhüter 501 eine Mehrzahl von Hinweisen auf Soll-Positionen 503, 505 angezeigt werden können.

Im modernen Fußballspiel soll der Torhüter hinter der hinteren Verteidigungskette als mitspielender Torhüter agieren. Als Grundregel gilt hierbei, dass der Torhüter sich auf der gedachten Verbindungslinie Tormitte-Ballmitte mitbewegen sollte. In einem Echtzeitfeedback-Trainings- bzw. Spielszenario könnte nun diese Verbindungslinie durch die Anzeigeeinrichtung 213 während des Trainings- bzw. Spielgeschehens permanent auf die Spiel- bzw. Trainingsfläche projiziert werden. Durch diese Echtzeitfeedback-Projektion könnten Torhüter sehr schnell lernen sich auf der gedachten Linie Tor-Ball zu bewegen. Neben der visuellen Rückmeldung könnte auch eine akustische und taktile Rückmeldung erfolgen falls die Soll-/Istwert Differenzen bestimmte Grenzwerte überschreiten. Der Trainer, bzw. Torhüter hat beispielweise die Möglichkeit, zu entscheiden in Bezug auf welche in der Datenbank hinterlegten statistischen Normwerte die Soll/Istwert Differenz berechnet werden soll. Hierzu werden in der Datenbank aus der Analyse von Videosequenzen des typischen Verhaltens eines bestimmten Torhüters Werte hinterlegt, die in an sich bekannter Weise gewonnen werden können. Die Sollwertposition des Torhüters wird beispielsweise so bestimmt, dass der durchschnittliche Abstand des Torhüters zum geometrischen Schwerpunkt der Mannschaft empirisch-statistsich zum Beispiel aus 100 typischen Videoszenen der Vergangenheit ermittelt und in der Datenbank hinterlegt wird. Dieser Sollwert wird nun mit dem permanent zu erfassenden Schwerpunkt des trainierenden oder spielenden Teams in eine mathematische Beziehung gesetzt und das Ergebnis in Echtzeit visuell während des Trainings oder Spiels dargestellt. So würde die Sollwertposition des Torhüters, die über die App 10 eingeschaltet werden kann in Form einer Kreisfläche (SWP 10) in Echtzeit dynamisch auf das Spiel- oder Trainingsfeld projiziert. Analog könnte auch das typische Verhalten eines anderen Torhüters in Echtzeit auf dem Platz dargestellt werden (SWP 11).

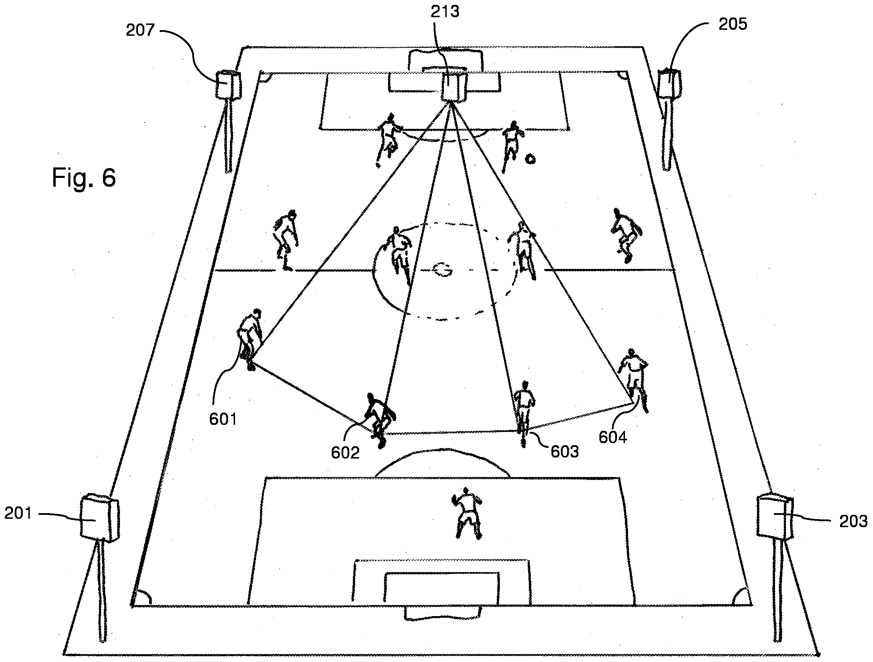

Fig. 6 zeigt ein System gemäß einer weiteren Ausführungsform, bei dem die Spieler 601- 604 Spielgegenstände im Sinne der vorliegenden Beschreibung sind. Dabei wird zumindest einem oder mehreren der Spieler 601 ein Hinweis auf dessen individuelle Soll-Position in Abhängigkeit von Ist-Positionen der übrigen Spieler angezeigt. Hierbei kann beispielsweise ein Schwerpunkt, beispielsweise ein geometrischer Schwerpunkt, aus den Ist-Positionen der übrigen Spieler bestimmt werden, um den jeweiligen Spieler 601 dessen individuelle Soll-Position anzuzeigen.

Gemäß einer Ausführungsform kann auf diese Weise der Abstand zwischen den Spielern überwacht werden. Der Abstand von Spieler 601 zu Spieler 602 ist beispielsweise zu groß, so dass ein Hinweis ausgegeben werden kann, diesen Abstand zu verkleinern, so dass er dem Soll-Abstand als Soll-Position entspricht. Der Soll-Abstand ist beispielsweise vorgegeben oder er ergibt sich aus Modellansätzen, die deterministisch und/oder empirisch-statistisch sein können, welche die Sollposition der Spieler in Echtzeit dynamisch verfügbar vorhalten.

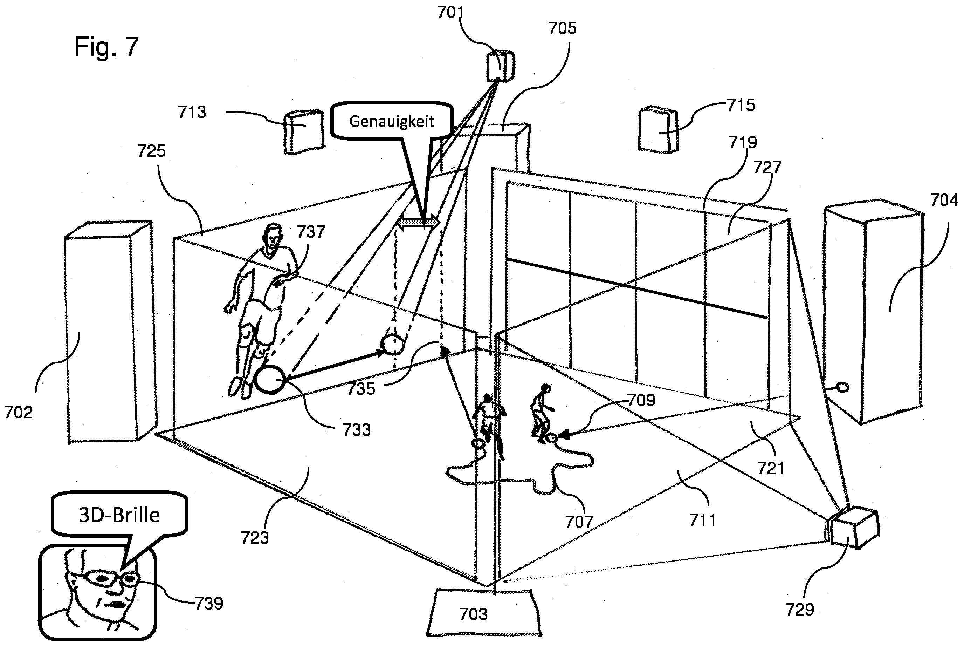

Fig. 7 zeigt ein System gemäß einer Ausführungsform, bei dem eine Bewegungsübung mit einem Ball, beispielsweise mit einem Fußball, unterstützt wird.

Das System umfasst zumindest eine Anzeigeeinrichtung 701, 729 und einen oder mehrere Ballgeber 702, 703, 704 und 705, welche jeweils ausgebildet sind, einen Ball 709 zum Spielfeld 711 hin auszugeben. Das System umfasst zumindest eine Bestimmungseinrichtung 713, 715, welche ausgebildet ist, eine Position des Balls 709 als Ist-Position des Balls zu erfassen. Hierzu kann der Ball 709 ausgebildet sein, ein Sendesignal auszusenden, das die zumindest eine Bestimmungseinrichtung 713, 715 zur Positionsbestimmung empfangen und in an sich bekannter Weise auswerten kann. Die zumindest eine Bestimmungseinrichtung 713, 715 kann insbesondere ausgebildet sein, eine Mehrzahl von Positionen des Balls 709 zu erfassen, welche in Fig. 7 durch die Trajektorie 707 beispielhaft dargestellt ist. Auf diese Weise kann zumindest eine Bestimmungseinrichtung 713, 715 eine Verweildauer des Balls 709 bei einem Spieler, welcher den Ball 709 annimmt und abgibt, erfassen. Die Verweildauer kann beispielsweise auf der Basis einer Abbremsung, d.h. einer negativen Beschleunigung, des Balls 709 zum Zeitpunkt der Ballannahme durch den Spieler sowie einer positiven Beschleunigung zum Zeitpunkt der Ballabgabe durch den Spieler erfassen.

Das System umfasst ferner zumindest einen Ballfänger 719, 721, 723, 725, welcher beispielsweise als eine Sensorwand ausgebildet ist, die das Spielfeld 711 zumindest teilweise begrenzt. Die Sensorwände 719, 721, 723 und 725 umfassen beispielsweise Sensorbereiche 727, welche mit Drucksensoren ausgestattet sein können. Hierzu kann eine Anzeigeeinrichtung 729 beispielsweise einen Bereich 727 anwählen, um eine Soll-Position des Balls 709 anzuzeigen. Dies kann beispielsweise durch ein Anleuchten des jeweiligen Bereichs 727 erfolgen. Auf diese Weise kann dem Spieler angezeigt werden, wohin er den Ball 709 schießen soll.

Gemäß einer zusätzlichen oder alternativen Ausführungsform ist die Anzeigeeinrichtung 701 vorgesehen, welche ausgebildet ist, eine Soll-Position 733 des Balls auf eine der Sensorwände 725 statisch oder dynamisch zu projizieren. Die Soll-Position kann beispielsweise, wie es in Fig. 7 dargestellt ist, veränderlich sein, was durch den Pfeil dargestellt ist. Die Sensorwand 725 kann nach der Ballabgabe einen Einschlagsort 735 erfassen, beispielsweise drucktechnisch, woraus eine Abweichung 737 zwischen der Soll-Position und der erfassten Position bestimmt werden kann. Der Einschlagsort 735 kann bei einer Lokalisation des Balles und einer dem Computer bekannten Raumgeometrie auch ohne Drucksensorik ermittelt werden. In diesem Fall kann auf die Drucksensorik bei der Sensorwand verzichtet werden.

Gemäß einer Ausführungsform kann die Anzeigeeinrichtung 701, 729 eine Spielfigur 737 auf zumindest eine der Sensorwände 719, 721, 723, 725 projizieren, um einen Mitspieler zu simulieren. Diese Simulation kann dreidimensional sein, wozu der Spieler beispielsweise mit dreidimensionalen Gläsern 739 ausgestattet werden kann, beispielsweise einer 3D-Brille.

Gemäß einer Ausführungsform ist ein Echtzeitlokalisationssystem von einem oder mehreren aktiven oder passiven Markern vorgesehen, das befestigt an einer oder mehreren Stellen eines oder mehrerer Personen und/oder eines oder mehrerer Spielgegenstande sein kann. Das Echtzeitlokalisationssystem umfasst beispielsweise zumindest eine Anzeigevorrichtung. Das Echtzeitlokalisationssystem kann ein Infrarotkinematographiesystem sein, dass mit passiven retroreflexiven Markern funktioniert, es kann aber auch ein videobasiertes Trackingsystem sein, oder ein funkbasiertes Lokalisationssystem, das mit aktiven Sendern und entsprechender Empfängertechnologie arbeitet. Es kann aber auch jedes andere aktive oder passive Lokalisationssystem sein.

Ferner kann ein Echtzeitbiosignalaufnahme- und Weiterleitungssystem zur synchronen Ableitung und Weiterleitung physiologischer Signale, z.B. Herzfrequenz, Herzfrequenzvariabilität, Köpertemperatur, Atemfrequenz, Hautleitwiederstand, Elektrolytzusammensetzung mit den vorstehend genannten Lokalisationsdaten vorgesehen sein. Darüber hinaus können technische Daten wie Luftdruck und/oder Rotation und/oder Beschleunigung berücksichtigt werden.

Ferner kann ein Datenbanksystem vorgesehen sein, das mit externen und/oder internen Daten gefüllt werden kann. Diese Datenbank wird permanent erweitert, entweder mit externen Daten oder internen Daten. Externe Daten sind zum Beispiel Daten aus Spielen oder Trainingseinheiten oder sonstigen Events, die per Video oder einer anderen Datenerfassungstechnik aufgezeichnet und analysiert worden sind. Die Datenbank nimmt auch Werte aus anderen Quellen auf. Zum Beispiel leistungsdiagnostische Kenngrößen aus Labor- und oder Feldstufentests oder auch anthropometrische Daten aus der Vermessung mit einem Bodyscanner, etc. Die Datenbank wird jedoch auch permanent und systematisch erweitert durch Daten, die aus den eigenen Aufnahmen (Istwerte) erfolgen. Diese Daten werden als interne Quellen bezeichnet.

Ferner kann ein Regelsystem vorgesehen sein, welches durch die Implementierung von Expertenwissen ständig erweitert wird. Experten können Sportwissenschaftler, Sportmediziner, Ingenieure, Fußballtrainer, etc. sein.

Ferner kann ein Setupmodul vorgesehen sein, das durch einen PC oder Tablet-PC oder Smartphone oder ein anderes Steuerungsgerät gesteuert wird. Dieses Setupmodul bestimmt, welche internen und externen Quellen für eine Trainings- Spiel oder Beobachtungseinheit zum Soll-Istwert Vergleich herangezogen werden. Ebenso wird über das Modul die Spezifikation für ein Echtzeitfeedbacksystem festgelegt.

Das Setupmodul, das Regelsystem und das Datenbanksystem können in der Bestimmungseinrichtung implementiert sein.

Ferner kann ein Echtzeitfeedbacksystem vorgesehen sein, das die Anzeigeeinrichtung umfasst und das Sollwerte und/oder Istwerte und/oder Soll-/Istwert Differenzen in Echtzeit an das Subjekt, bzw. die Subjekte in Form von optisch/visuellen und/oder akustischen und/oder taktilen Signalen sendet. Visuelle Signale können sein: ein Laserprojektionssystem oder eine anderes optisches Projektionssystem oder einen Bildschirm oder ein anderes optisches/visuelles Darstellungsverfahren, ein akustisches System, umfassend einen Miniaturkopfhörer oder eine Beschallungsanlage oder ein am Körper befindliches Lautsprechersystem, oder zumindest ein Vibrationsgerät, das am Körper an definierten Stellen getragen werden kann.

## Patentansprüche

1. System zum Erfassen eines benutzerabhängigen Zustandes eines Sportgegenstandes, mit:
einer Erfassungseinrichtung (101) zum Erfassen einer Mehrzahl von Positionen des Sportgegenstandes;
einer Bestimmungseinrichtung (103) zum Bestimmen des Zustandes des Sportgegenstandes auf der Basis der erfassten Mehrzahl der Positionen; und
einer Anzeigeeinrichtung (105) zum Anzeigen der erfassten Mehrzahl der Positionen;
wobei die Erfassungseinrichtung (101) zum Erfassen einer Ist-Position des Sportgegenstandes ausgebildet ist, wobei die Bestimmungseinrichtung (103) zum Bestimmen einer Soll-Position des Sportgegenstandes ausgebildet ist, und wobei die Anzeigeeinrichtung (105) zum Anzeigen eines Hinweises auf die Soll-Position ausgebildet ist, wenn sich die Ist-Position von der Soll-Position unterscheidet,
**gekennzeichnet dadurch, dass** der Sportgegenstand ein Ball oder ein Puck ist.

2. System nach Anspruch 1, wobei der Zustand des Sportgegenstandes einer der folgenden Zustände ist: Verweildauer des Sportgegenstandes bei einem Benutzer, Geschwindigkeit, insbesondere translatorische Geschwindigkeit oder Rotationsgeschwindigkeit des Sportgegenstandes, Impuls, insbesondere Drehimpuls oder translatorischer Impuls des Sportgegenstandes, Bewegungsrichtung des Sportgegenstandes, Beschleunigung, insbesondere positive oder negative Beschleunigung des Sportgegenstandes.

3. System nach einem der vorstehenden Ansprüche, wobei der Sportgegenstand ausgebildet ist, ein Positionssignal auszusenden, und wobei die Erfassungseinrichtung ausgebildet ist, das Positionssignal zu empfangen, um die Mehrzahl der Positionen zu bestimmen.

4. System nach einem der vorstehenden Ansprüche, wobei die Erfassungseinrichtung (101) ausgebildet ist, ein an dem Sportgegenstand reflektiertes Reflexionssignal, insbesondere ein Radarsignal oder ein Lasersignal, zu empfangen, und auf der Basis des reflektierten Reflexionssignals die Mehrzahl der Positionen zu bestimmen.

5. System nach einem der vorstehenden Ansprüche, wobei die Bestimmungseinrichtung (103) ausgebildet ist, einen ersten Zeitpunkt einer Annahme des Sportgegenstandes durch den Benutzer und einen zweiten Zeitpunkt einer Abgabe des Sportgegenstandes durch den Benutzer zu bestimmen, um eine Verweildauer des Sportgegenstandes bei dem Benutzer als den benutzerabhängigen Zustand des Sportgegenstandes zu bestimmen.

6. System nach Anspruch 5, wobei die Bestimmungseinrichtung (103) ausgebildet ist, einen dritten Zeitpunkt eines Umlegens des Sportgegenstandes durch den Benutzer zwischen dem ersten Zeitpunkt und dem zweiten Zeitpunkt zu bestimmen, um eine erste Unterverweildauer zwischen dem ersten und dem dritten Zeitpunkt und eine zweite Unterverweildauer zwischen dem dritten und dem zweiten Zeitpunkt zu bestimmen.

7. System nach einem der vorstehenden Ansprüche, wobei die Bestimmungseinrichtung (103) ausgebildet ist, zur Bestimmung des benutzerabhängigen Zustandes einen Zeitpunkt einer Annahme des Sportgegenstandes durch den Benutzer durch eine Erfassung einer negativen Beschleunigung des Sportgegenstandes anhand eines zeitlichen Verlaufes der Mehrzahl der Positionen des Sportgegenstandes zu bestimmen.

8. System nach einem der vorstehenden Ansprüche, wobei die Bestimmungseinrichtung (103) ausgebildet ist, zur Bestimmung des benutzerabhängigen Zustandes einen Zeitpunkt einer Abgabe des Sportgegenstandes durch eine Erfassung einer positiven Beschleunigung des Sportgegenstandes anhand eines zeitlichen Verlaufes der Mehrzahl der Positionen des Sportgegenstandes zu bestimmen.

9. System nach einem der vorstehenden Ansprüche, die ferner einen Sportgegenstandgeber, insbesondere einen Ballgeber, zum Ausgeben des Sportgegenstandes umfasst.

10. System nach einem der vorstehenden Ansprüche, die ferner einen Sportgegenstandfänger zum Fangen des Sportgegenstandes nach einer Sportgegenstandabgabe durch den Benutzer umfasst.

11. System nach Anspruch 10, wobei der Sportgegenstandfänger ein Balltor oder eine Sensorwand ist.

12. System nach einem der vorstehenden Ansprüche, wobei die Erfassungseinrichtung (101) ausgebildet ist, die erfasste Mehrzahl der Positionen zu speichern.

13. System nach einem der vorstehenden Ansprüche, wobei die Anzeigeeinrichtung (105) zum Projizieren eines Projektionsbereiches auf ein Spielfeld ausgebildet ist.

14. System nach einem der vorstehenden Ansprüche, wobei die Bestimmungseinrichtung (103) ausgebildet ist, den benutzerabhängigen Zustand mit einem Referenzzustand zu vergleichen, um den benutzerabhängigen Zustand zu bewerten.

15. System nach einem der vorstehenden Ansprüche, wobei die Bestimmungseinrichtung (103) ausgebildet ist, die Soll-Position in Abhängigkeit von der Ist-Position des Spielgegenstandes zu bestimmen.

16. System nach Anspruch 15, wobei die Erfassungseinrichtung (101) ausgebildet ist, eine Mehrzahl von weiteren Ist-Positionen von einer Mehrzahl von weiteren Spielgegenständen zu bestimmen, wobei die Bestimmungseinrichtung (103) ausgebildet ist, die Soll-Position des Spielgegenstandes ferner in Abhängigkeit von der Mehrzahl der weiteren Ist-Positionen zu bestimmen.

17. System nach einem der vorstehenden Ansprüche, wobei die Anzeigeeinrichtung (105) ausgebildet ist, als Hinweis auf die Soll-Position eine Differenz zwischen der Ist-Position und der Soll-Position anzuzeigen.

18. Verfahren zum Erfassen eines benutzerabhängigen Zustandes eines Sportgegenstandes, mit:
Erfassen einer Mehrzahl von Positionen des Sportgegenstandes;
Bestimmen des Zustandes des Sportgegenstandes auf der Basis der erfassten Mehrzahl der Positionen;
Anzeigen der erfassten Mehrzahl der Positionen;
Erfassen einer Ist-Position des Sportgegenstandes;
Bestimmen einer Soll-Position des Sportgegenstandes; und
Anzeigen eines Hinweises auf die Soll-Position, wenn sich die Ist-Position von der Soll-Position
unterscheidet,
**gekennzeichnet dadurch, dass** der Sportgegenstand ein Ball oder ein Puck ist.

## Claims

1. System for detecting a user-dependent state of a sports object, comprising:
a detection device (101) for detecting a plurality of positions of the sports object;
a determination device (103) for determining the state of the sports object on the basis of the detected plurality of positions; and
an indication device (105) for indicating the detected plurality of positions wherein the detection device (101) is designed for detecting an actual position of the sports object, wherein the determination device (103) is designed for determining a desired position of the sports object, and wherein the indication device (105) is designed for indicating information relating to the desired position if the actual position differs from the desired position, **characterized in that** the sports object is a ball or a puck.

2. System according to Claim 1, wherein the state of the sports object is one of the following states:
residence duration of the sports object at a user, velocity, in particular translational velocity or rotational velocity, of the sports object, momentum, in particular angular momentum or translational momentum, of the sports object, direction of movement of the sports object, acceleration, in particular positive or negative acceleration, of the sports object.

3. System according to any of the preceding claims, wherein the sports object is designed to emit a position signal, and wherein the detection device is designed to receive the position signal in order to determine the plurality of positions.

4. System according to any of the preceding claims, wherein the detection device (101) is designed to receive a reflection signal reflected at the sports object, in particular a radar signal or a laser signal, and to determine the plurality of positions on the basis of the reflected reflection signal.

5. System according to any of the preceding claims, wherein the determination device (103) is designed to determine a first instant of an acceptance of the sports object by the user and a second instant of a release of the sports object by the user, in order to determine a residence duration of the sports object at the user as the user-dependent state of the sports object.

6. System according to Claim 5, wherein the determination device (103) is designed to determine a third instant of a transfer of the sports object by the user between the first instant and the second instant, in order to determine a first sub-residence duration between the first instant and the third instant and a second sub-residence duration between the third instant and the second instant.

7. System according to any of the preceding claims, wherein the determination device (103) is designed, for the purpose of determining the user-dependent state, to determine an instant of an acceptance of the sports object by the user by detection of a negative acceleration of the sports object with the aid of a temporal profile of the plurality of positions of the sports object.

8. System according to any of the preceding claims, wherein the determination device (103) is designed, for the purpose of determining the user-dependent state, to determine an instant of a release of the sports object by detection of a positive acceleration of the sports object with the aid of a temporal profile of the plurality of positions of the sports object.

9. System according to any of the preceding claims, which furthermore comprises a sports object provider, in particular a ball provider, for dispensing the sports object.

10. System according to any of the preceding claims, which furthermore comprises a sports object catcher for catching the sports object after a sports object release by the user.

11. System according to Claim 10, wherein the sports object catcher is a ball goal or a sensor wall.

12. System according to any of the preceding claims, wherein the detection device (101) is designed to store the detected plurality of positions.

13. System according to any of the preceding claims, wherein the indication device (105) is designed for projecting a projection region onto a playing area.

14. System according to any of the preceding claims, wherein the determination device (103) is designed to compare the user-dependent state with a reference state in order to assess the user-dependent state.

15. System according to any of the preceding claims, wherein the determination device (103) is designed to determine the desired position in a manner dependent on the actual position of the playing object.

16. System according to Claim 15, wherein the detection device (101) is designed to determine a plurality of further actual positions from a plurality of further playing objects, wherein the determination device (103) is designed to determine the desired position of the playing object furthermore in a manner dependent on the plurality of further actual positions.

17. System according to any of the preceding claims, wherein the indication device (105) is designed to indicate as information relating to the desired position a difference between the actual position and the desired position.

18. Method for detecting a user-dependent state of a sports object, comprising:
detecting a plurality of positions of the sports object;
determining the state of the sports object on the basis of the detected plurality of positions;
indicating the detected plurality of positions;
detecting an actual position of the sports object;
determining a desired position of the sports object; and
indicating information relating to the desired position if the actual position differs from the desired position,
**characterized in that** the sports object is a ball or a puck.

## Revendications

1. Système pour détecter un état dépendant de l'utilisateur d'un article de sport, comportant :
un dispositif de détection (101) pour détecter une pluralité de positions de l'article de sport ;
un dispositif de détermination (103) pour déterminer l'état de l'article de sport sur la base de la pluralité de positions détectées ; et
un dispositif d'affichage (105) pour afficher la pluralité de positions détectées ;
dans lequel le dispositif de détection (101) est conçu pour détecter une position réelle de l'article de sport, dans lequel le dispositif de détermination (103) est conçu pour déterminer une position de consigne de l'article de sport, et dans lequel le dispositif d'affichage (105) est conçu pour afficher une indication de la position de consigne lorsque la position réelle est différente de la position de consigne,
**caractérisé en ce que** l'article de sport est un ballon ou un palet.

2. Système selon la revendication 1, dans lequel l'état de l'article de sport est l'un des états suivants : une durée de maintien de l'article de sport par un utilisateur, une vitesse, en particulier la vitesse de translation ou la vitesse de rotation de l'article de sport, une impulsion, en particulier une impulsion de rotation ou une impulsion de translation de l'article de sport, une direction de déplacement de l'article de sport, une accélération, en particulier une accélération positive ou négative de l'article de sport.

3. Système selon l'une des revendications précédentes, dans lequel l'article de sport est conçu pour transmettre un signal de position, et dans lequel le dispositif de détection est conçu pour recevoir le signal de position afin de déterminer la pluralité de positions.

4. Système selon l'une des revendications précédentes, dans lequel le dispositif de détection (101) est conçu pour recevoir un signal de réflexion, en particulier un signal radar ou un signal laser, réfléchi par l'article de sport, et pour déterminer la pluralité de positions sur la base du signal réfléchi.

5. Système selon l'une des revendications précédentes, dans lequel le dispositif de détermination (103) est conçu pour déterminer un premier instant de prise de l'article de sport par l'utilisateur et un deuxième instant d'envoi de l'article de sport par l'utilisateur pour déterminer une période de maintien de l'article de sport par l'utilisateur en tant qu'état dépendant de l'utilisateur pour l'article de sport.

6. Système selon la revendication 5, dans lequel le dispositif de détermination (103) est conçu pour déterminer un troisième instant où l'utilisateur déplace l'article de sport entre le premier instant et le deuxième instant afin de déterminer une première durée de maintien secondaire entre le premier et le troisième instant et une deuxième durée de maintien secondaire entre le troisième et le deuxième instant.

7. Système selon l'une des revendications précédentes, dans lequel le dispositif de détermination (103) est conçu pour déterminer un instant de prise de l'article de sport par l'utilisateur en détectant une accélération négative de l'article de sport sur la base d'une évolution dans le temps des différentes positions de l'article de sport afin de déterminer l'état dépendant de l'utilisateur.

8. Système selon l'une des revendications précédentes, dans lequel le dispositif de détermination (103) est conçu pour déterminer un instant d'envoi de l'article de sport en détectant une accélération positive de l'article de sport sur la base d'une évolution dans le temps de la pluralité de positions de l'article de sport, afin de déterminer l'état dépendant de l'utilisateur.

9. Système selon l'une des revendications précédentes, qui comprend en outre un donneur d'article de sport, en particulier un donneur de ballon, pour l'envoi de l'article de sport.

10. Système selon l'une des revendications précédentes, qui comprend en outre un receveur d'article de sport destiné à intercepter l'article de sport après l'envoi de l'article de sport par l'utilisateur.

11. Système selon la revendication 10, dans lequel le receveur d'article de sport est un but pour ballons ou un mur capteur.

12. Système selon l'une des revendications précédentes, dans lequel le dispositif de détection (101) est conçu pour stocker la pluralité de positions détectées.

13. Système selon l'une des revendications précédentes, dans lequel le dispositif d'affichage (105) est conçu pour projeter une zone de projection sur un terrain de jeu.

14. Système selon l'une des revendications précédentes, dans lequel le dispositif de détermination (103) est conçu pour comparer l'état dépendant de l'utilisateur avec un état de référence afin d'évaluer l'état dépendant de l'utilisateur.

15. Système selon l'une des revendications précédentes, dans lequel le dispositif de détermination (103) est conçu pour déterminer la position de consigne en fonction de la position réelle de l'article de jeu.

16. Système selon la revendication 15, dans lequel le dispositif de détection (101) est conçu pour déterminer une pluralité d'autres positions réelles d'une pluralité d'autres articles de jeu, dans lequel le dispositif de détermination (103) est conçu pour déterminer en outre la position de consigne de l'article de jeu en fonction de la pluralité d'autres positions réelles.

17. Système selon l'une des revendications précédentes, dans lequel le dispositif d'affichage (105) est conçu pour afficher une différence entre la position réelle et la position de consigne en tant qu'indication de la position de consigne.

18. Procédé pour détecter un état dépendant de l'utilisateur d'un article de sport, comprenant :
la détection d'une pluralité de positions de l'article de sport ;
la détermination de l'état de l'article de sport sur la base de la pluralité de positions détectées ;
l'affichage de la pluralité détectée de positions ;
la détection d'une position réelle de l'article de sport ;
la détermination d'une position de consigne de l'article de sport ; et
l'affichage d'une indication de la position de consigne lorsque la position réelle est différente de la position de consigne,
**caractérisé en ce que** l'article de sport est un ballon ou un palet.
